Europäisches Patentamt

European Patent Office

Office européen des brevets

(11) Publication number: **0 347 501**
**A1**

## (12) EUROPEAN PATENT APPLICATION

(21) Application number: 88305787.9

(51) Int. Cl.4: **A61K 31/70 , C07H 21/00**

(22) Date of filing: **22.06.88**

(43) Date of publication of application:
**27.12.89 Bulletin 89/52**

(84) Designated Contracting States:
**AT BE CH DE ES FR GB GR IT LI LU NL SE**

(71) Applicant: **HEM RESEARCH, INC.**
**12280 Wilkins Avenue P.O. Box 2245**
**Rockville, MD 20852(US)**

(72) Inventor: **Carter, William Alvin**
**1 Jaine Lane**
**Birchrunville Pennsylvania 19421(US)**

(74) Representative: **Votier, Sidney David et al**
**CARPMAELS & RANSFORD 43, Bloomsbury**
**Square**
**London WC1A 2RA(GB)**

(54) Modulation of lymphokine-resistant cellular states by dsRNAs.

(57) Tumor cells sub- or non-responding to the antiproliferative effects of lymphokines, notably the interferons and the interleukins, and rendered lymphokine-responsive by exposing the tumor cells to exogenous mismatched dsRNAs. These dsRNAs themselves modulate cell growth of susceptible tumors towards normalization and loss of cancerous cell phenotype.

EP 0 347 501 A1

## MODULATION OF LYMPHOKINE-RESISTANT CELLULAR STATES BY dsRNAs

### FIELD OF THE INVENTION

This invention relates to the use of double stranded ribose nucleic acid (hereinafter referred to as dsRNA) alone or in combination with lymphokines, to inhibit the proliferation of neoplastic cells in an animal body, including humans, to correct defects present in tumorous or cancerous cells in an animal body, including humans, and to treat immune system disorders stemming from the predisposition of certain individuals to be susceptible to tumorous or cancerous conditions, whether this predisposition arises from either genetic (hereditary) or environmental causes. The invention further relates to the use of dsRNAs in combination with other chemical and biological agents to treat tumorous or cancerous conditions.

### BACKGROUND OF THE INVENTION

#### I. IFN Functions and dsRNAs as IFN Inducers

Interferons (hereinafter IFNs) constitute a class of proteins capable of inhibiting virus replication in animal cells. They are of cellular origin, may be produced in vitro or in vivo, and are stimulated by a fairly wide range of materials (The Encyclopedia of Biochemistry, edited by Roger Williams and Edwin Lansford, Reinhold Publishing Company, 1967). IFN is known to exert antiproliferative effects as well as antiviral effects (Nature 262, 300, 1978), to reestablish contact inhibition (J. Cell Biol., 56 846, 1973), and to inhibit the growth in semi-solid agar of neoplastic cells (Nature, 231, 20, 1971).

dsRNAs are inducers of the different molecular forms of human IFN including alpha (leukocyte), beta (fibroblast), and gamma (immune) types (J. Reticuloendothelial Society, 23, 299, 1978) with IFN production capable of being stimulated by both natural and synthetic dsRNAs. The use of synthetic dsRNA in the form of complexes of polyriboinosinic and polyribocytidilic acids (hereinafter referred to as $rI_n.rC_n$ or Poly IC) strictly as an interferon inducer is known, for example, from U.S. Patent No. 3,666,646 issued to Lampson et al. In addition to its role of IFN induction, dsRNA is also an activator of two IFN-induced enzymes, a protein kinase and a $2'-5'$ oligoadenylate synthetase (Proc. Natl. Acad. Sci., 75, 5893, 1978). The molecular bases for the antiproliferative and antineoplastic effects of interferon and their relationship to dsRNA are, however, unknown, and prior to the present invention, any antiproliferative effects of dsRNA beyond its role as in interferon inducer were also unknown.

#### II. Synthetic Mismatched Analogs of dsRNA

More recently, it has been discovered that IFN can also be induced by mismatched analogs of $rI_n \cdot rC_n$, as disclosed in U.S. Patent Nos. 4,130,641 and 4,024,222 issued to Ts'o and Carter. These analogs are formed by modifying $rI_n \cdot rC_n$ to incorporate unpaired bases (uracil or guanine) along the polyribocytidylate $(rC_n)$ strand or by modifying the ribosyl backbone of polyriboinosinic acid $(rI_n)$. The mismatched analogs, particularly those modified in the $rC_n$ strand, retain the ability to induce interferon so long as certain structural requirements are met. Long regions of perfectly base paired dsRNA are not required for interferon induction. In fact, the structural prerequisite for triggering interferon synthesis appears to be the preservation of 1/2 to 1 helical turn of perfectly base paired dsRNA.

An accelerated rate of hydrolysis has been noted when the mismatched polymers were exposed to nucleases (J. Molec. Biol., 70, 567, 1972), yet these dsRNAs were nearly as active as unmodified $rI_n \cdot rC_n$ in terms of interferon induction. It was proposed that prolonged maintenance of an intact double helical structure might be responsible for the many other biological responses to dsRNA commonly scored as drug toxicities. Studies of the mismatched analog, $rI_n \cdot r(C_{11-14})_n$, Ampligen®, a registered U.S. trademark of HEM Research, Inc., Rockville, Md., 10852, USA, in various animal systems have shown $rI_n \cdot r(C_{12},U)_n$ to be less toxic than $rI_n \cdot rC_n$ (see Poly IC with mismatched bases, prospects for cancer therapy, in Augmenting Agents in Cancer Therapy, edited by E.M. Hersh et al, Raven Press, New York, 177, 1981). In particular, repeated administration of $rI_n \cdot r(C_{12},U)_n$ to mice results in much less toxicity to critically sensitive organs

2

including bone marrow elements, liver cells, thymocytes and splenocytes (J. Molec. Biol., 70, 567, 1972), while effectiveness in protecting against a variety of lethal viral challenges is preserved (Molec. Pharm., 12 299, 1976).

Additionally, $rI_n \cdot r(C_{11-14}, U)_n$ exhibits reduced (100-fold reduction) pyrogenicity relative to $rI_n \cdot rC_n$ in rabbits, lower mitogenicity (5 to 10 fold reduction) with mouse and human cells in vitro, and decreased dsRNA antibody formation (5 to 10 fold reduction) in rabbits and mice. Against this background of reduced cellular toxicity, $rI_n \cdot r(C_{11-14}, U)_n$ preserves its ability to induce interferon, stimulate natural killer (NK) cell function (J. Immuno, 124, 1852, 1980), and activate the above mentioned intracellular mediators, 2'-5' oligo-adenylate synthetase and a specific protein kinase. It is therefore clear that, in a variety of test systems (rabbit, mouse, and man) and under different dose schedules measuring various toxicities and therapeutic effects, the mismatched analog, $rI_n \cdot (C_{11-14}, U)_n$ (Ampligen®) has an enhanced therapeutic ratio. The preparation and formulation of this mismatched inducer/activator has been described (J. Molec. Bio. 70, 567, 1972).

The use of modified $rI_n \cdot rC_n$ analogs for the sole purpose of inducing interferon production in animal cells to combat viral attack is known, for example, from the aforementioned U.S. Patent Nos. 4,130,641 and 4,024,222. However, with respect to its use as an antiproliferative agent, an impediment to IFN (as well as other lymphokines administered alone) therapy exists in that such lymphokines exhibit only a marginal effect in many individuals. Consequently, relatively large amounts of lymphokines are required for administration, resulting in unnaturally high bodily lymphokine levels that would not be generated in the course of normal human response to an ordinary viral infection, immune cell differentiation or natural immunosurveillant mechanisms pertinent to anticancer, antiviral and host defense mechanisms. At these high levels, the body can treat the lymphokine such as IFN as a foreign substance, and treated individuals have the capability to generate antibodies against various lymphokines such as IFN. These resultant antibodies generally destroy any residual therapeutic benefits.

Moreover, neoplastic cells in particular have the capability to acquire resistance to the therapeutic properties of various lymphokines such as IFN and the different members of the interleukin family. As it will be demonstrated below, this ability of neoplastic cell lines to develop resistance against IFN is acquired as a non-random phenotype, and it therefore poses a severe drawback to any therapeutic method designed to inhibit proliferation predicated on the use of lymphokines alone. The present invention remedies this prior art deficiency by disclosing novel antiproliferative agents and compositions which halt or dramatically retard increases in cancerous cells, whether or not the cells have developed lymphokine resistance. Moreover, this invention provides compositions containing interferon in which the bodily levels of interferon, although lower by a factor of 20 to 50 than the levels achieved by the prior art, result nonetheless in an even greater therapeutic effectiveness. Because the compositions of this invention containing dsRNA act synergistically, the therapeutic benefits of dsRNA are similarly much higher than those disclosed by the prior art and indeed provide therapeutic attack of tumor nests in sanctuaries of bone matrix, which tumor sanctuaries are notoriously resistant to attach to exogenously applied lymphokines when they are given as mono-modality therapy.

III. Natural Killer (NK) Cells as an Immunosurveillance Mechanism Against Tumor Cells

Numerous papers have been published which support a central role for NK cell populations in immunosurveillance mechanisms against neoplastic cells. It has been shown that mice with high NK-cell activity were more resistant to the growth of NK-sensitive tumor cells than were mice with low NK-cell activity (Immunol. Rev., 44, 165, 1979). It was also reported that C57BL/6 beige mice, which have a selective NK-cell defect, are more susceptible to tumor growth than are syngeneic mice with normal NK-cell activity (Nature (Lond.), 284, 622, 1980; Nature, 284, 624, 1980). A similar condition exists in humans with Chediak-Higashi syndrome and it has been suggested that the NK-cell defect in this disease may be causally related to the subsequent development of lymphomas in these patients (J. EXP. Med., 151, 1039, 1980; J. EXP. Med., 151, 1049, 1980); Nature (Lond.), 284, 553, 1980). Patients with chronic lymphocytic leukemia (CLL) are known to have a high incidence of secondary malignancies. Ziegler and Zarling (Int. J. Cancer, 27, 321, 1981) assessed NK cell activity in lymphocytes from chronic leukemia patients following removal of the leukemic cells. Significantly, the majority of CLI patients have minimal or no detectable NK cell activity despite the presence of lymphocytes which bind to NK-sensitive targets. In addition, renal allograft recipients, which are treated with high-dose immunosuppressive drugs, have an approximately 100-fold higher risk of developing malignancies and also have extremely depressed or abolished NK cell activities (Transplantation, 29, 214, 1980). In contrast, antibody-dependent cell mediated cytotoxicity (ADCC)

EP 0 347 501 A1

is not as depressed in these recipients. This suggests that the defect in immune cell activity is a specific one and, when present, is associated with a profound propensity for the development/recurrence of malignancy.

## SUMMARY OF THE INVENTION

The present invention is based on multiple discoveries including the observation that even though tumor cells may be IFN-resistant, the use of specific lymphokines in combination with mismatched dsRNA results in a synergistic or potentiated effect with respect to that antiproliferative action. That is, the use of mismatched dsRNA alone or in combination with lymphokines in individuals who exhibit only marginal response to single modality lymphokine therapy results in an enhanced antiproliferative/immunomodulatory effect of mismatched dsRNA beyond that which results from therapy involving the use of mismatched dsRNA alone.

Additionally, for many individuals, lymphokines, e.g., IFN, therapy constitutes a feasible method of treatment. That is, these individuals do not require abnormally large dosage levels of IFN and, consequently, they run a much small risk of developing antibodies against lymphokines or sustaining other side effects of the administered lymphokine, these side-effects being related directly to lymphokine dosage. In these subjects, dsRNAs potentiate the action of lymphokines and thereby render IFN therapy an even more efficacious therapeutic method. Therefore, the therapeutic methods and compositions herein disclosed improve existing treatment methods based on lymphokines alone as well as provide access to these treatment methods to those individuals who would otherwise be precluded from efficacious treatment for one or more of these reasons.

A preferred embodiment of the present invention is when the dsRNA is a mismatched analog of $rI_n \cdot rC_n$. As previously mentioned, these mismatched analogs result from the interruption of either strand with unpaired bases (i.e., that do not form Watson-Crick base pairs) such as uracil or guanine. These mismatched analogs represent preferred embodiments due to the fact that they retain the antiproliferative effects of the unmodified $rI_n \cdot rC_n$ while displaying a greatly reduced tendency to trigger undesirable side effects such as fever, non-specific cell death (that is, the death of normal body cells), and antigenicity.

Moreover, I have also discovered that the specific mismatched analog, $rI_n \cdot r(C_{11-14}, U)_n$ exhibits dramatic effects even beyond those noted above in connection with the antiproliferative capabilities of a typical dsRNA such as $rI_n \cdot rC_n$. Specifically, it allows for the capability in some cases to correct defects in tumor cells such that they are reprogrammed and functionally converted to normal. I have in fact isolated cancer cells from patients and repeatedly observed this phenomenon. In addition, I have discovered that Ampligen can effectively function as a cancer cell normalizing agent in situations where lymphokines actually poses a complete obstacle to said treatment. Certain chemical and biological substances are known to possess some ability to independently normalize cancer cells. Mismatched dsRNAs, notably $rI_n \cdot r(C_{11-14}, U)$, is able to amplify this type of tumor cell normalizing activity while certain lymphokines, especially lymphokines, often have the opposite effect, i.e., they reduce or nullify this normalization process.

Moreover, the present invention further allows for the correction of immune disorders in individuals predisposed to developing cancer by virtue of family background (i.e., heredity) or defects in the immune system. This capability is apparently a non-IFN, non-lymphokine effect, and evidences the fact that mismatched dsRNAs can affect cancerous cells in a qualitative way that lymphokines alone do not.

The therapeutic compositions and methods provide for the treatment of cancer, tumors, and neoplastic cells. "Treatment", in this context, is a generic term encompassing the prevention of cancer, tumor arrest (whether partial or complete), the failure of tumors to reappear in patients previously treated by established therapy, the conversion of tumor cells to normal, and the correction of cancer-related immune defects and enhancement of host defense necessary to provide protection against viral infections as well as various autoimmune/inflammatory states. Importantly, it is crucial to understand that this invention applies not only to the treatment of cancer or tumors once they have become clinically manifest, but also to the prevention or abortion of these malignancies while they are incipient or subclinical. That is, cancerous conditions may be subclinical for as long as decades before becoming overtly apparent and thereby becoming subject to existing therapeutic methods. The invention, on the other hand, provides for averting cancer before it ever reaches a clinically apparent stage, for example, in individuals having backgrounds indicating a high predisposition to cancer. Viewed from a different perspective, cancer is simply part of a (potentially decades long) biological continuum wherein the early stages of the continuum may be simply a predisposi-

4

tion. The invention provides not only for the correction of overtly apparent cancer, but also allows for the prevention of cancer if it is treated during the early stages of this continuum, possibly decades before it actually appears.

It is therefore an object of this invention to provide therapeutic methods and compositions useful for the treatment of cancer in animals, including humans, "treatment" (when used in conjunction with cancer, tumor, etc.) being a generic term encompassing the prevention of cancer, tumor arrest (whether partial or complete), the failure of tumors to reappear in patients previously treated by established therapy, the conversion of tumor cells to normal, or the correction of cancer related immune defects and enhancement in host defense necessary to provide protection against viral infections as well as various autoimmune/inflammatory states.

It is a further object of this invention to provide improved therapeutic methods for inhibiting the proliferation of neoplastic cells in animals, including humans, comprising administering a mismatched dsRNA to animals in need of such therapeutic treatment.

It is a further object of this invention to provide improved therapeutic methods for inhibiting the proliferation of IFN-resistant neoplastic cells in animals in need of such therapeutic treatment.

It is a further object of this invention to provide methods and compositions for potentiating the antiproliferative effects of dsRNA on neoplastic cells in an animal body, including humans, comprising administering a mismatched dsRNA to an animal in combination with a lymphokine.

It is a further object of this invention to provide methods and compositions for the treatment of tumors, including those tumors resistant to IFN alone, in an animal body, including humans, comprising administering a mismatched dsRNA alone or in combination with a lymphokine.

It is a further object of this invention to provide methods whereby cancer or tumor cells in an animal body, including humans, may be functionally converted to normal comprising administering a mismatched dsRNA to the animal.

It is a further object of this invention to provide methods and compositions whereby cancer or tumor cells in an animal body may be functionally converted to normal, comprising administering a mismatched dsRNA to an animal, including humans, in combination with another chemical or biological substance which independently possesses some capability to normalize neoplastic cells.

It is a further object of this invention to provide methods whereby immune and chronic viral disorders can be ameliorated or arrested comprising, administering an effective amount of a mismatched dsRNA to such individuals.

## BRIEF DESCRIPTION OF THE DRAWINGS

The invention is further demonstrated with reference to the appended drawings in which:

FIGURE 1A is a graph reporting the indirect measurement of the induction of cAMP levels by measuring the adenylate catalyse activity in human glioma cells treated with mismatched dsRNA (two concentrations) over 30 minutes;

FIGURE 1B is a graph similar to Figure 1A reporting similar results over a period of 24 hours;

FIGURE 2A is a graph reporting the direct measurement of the induction of cAMP levels by RIA measurement in human glioma cells treated with mismatched dsRNA (two concentrations) over 30 minutes;

FIGURE 2B is a graph similar to Figure 2A reporting similar results over 24 hours;

FIGURE 3 is a bar graph reporting protein synthesis in human glioma cells (untreated, IFN-treated, dsRNA treated and both IFN and dsRNA-treated) over 24, 48 and 72 hours with progressive differentiation and loss of malignant cell phenotype, a non-lymphokine property, for dsRNA-treated cells;

FIGURE 4 is a graph of the results of a 31 day experiment measuring tumor size (length times width) of tumors engrafted in athymic mice as between no (control), IFN and dsRNA treatments;

FIGURE 5 is a graph of the results of colonogenic assay in melonoma cells for increasing amounts of IFN-alpha, IFN-beta and dsRNA;

FIGURE 6 is a graph of the results of a cologenic assay in human renal cell carcinoma for IFN-alpha alone and two combinations of IFN-alpha at zero and 100, 300 and 1000 units with 50 and 100 μg/ml of the dsRNA demonstrating synergistic antiproliferative effects for the combinations;

FIGURE 7 reports the response of a malignant melanoma patient in terms of tumor volume and $2',5'$-adenylate synthetase activity before and after more than 75 days of dsRNA therapy;

FIGURE 8 is a series of four HPLC graphs measuring the presence of various oligomers, especially $P_3A_3$, before and after initiation of dsRNA therapy in the patient of Figure 7;

5

FIGURE 9 i s an illustration of elevated LAK activity in a stabilized carcinoid cancer following more than 30 months dsRNA therapy as compared with LAK activity for solid tumor patients not receiving dsRNA therapy and normal patients as controls, and is used to monitor the amounts of dsRNA maintenance therapy required to sustain a favorable clinical effect over an extended period of time; and

FIGURE 10 reports the response of an adenocarcinoma lung tumor patient, in terms of NK cell activity and mediastinal tumor size before and following almost 400 days of mismatched dsRNA therapy.

## SUMMARY OF THE INVENTION

Procedures and therapeutic compositions for modulating tumor cell growth towards cell normalization and loss of malignant cell phenotype by the administration of exogenous mismatched dsRNA to the tumor cells are described, such as administering the mismatched dsRNA to the patient and increasing the patient's intracellular dsRNA level. Tumor cells not responding or sub-responding to the lymphokine are thus rendered responsive. The dsRNA may be continued concurrently with the lymphokine administration. Particularly dramatic responses are achieved in rendering indolent tumor cells responsive to the antiproliferative effects of interferons.

## DESCRIPTION OF PREFERRED EMBODIMENTS

As previously stated, many individuals do not respond satisfactorily to cancer therapy based on the use of IFN alone. Consequently, high dosage levels are required with the result that such individuals are actually able to generate resistance to their natural bodily product, IFN. Moreover, the fact that resistance of another kind can also be developed by neoplastic cells as a non-randomly acquired phenotype is a consideration of paramount importance in understanding the dramatic improvement which the therapeutic compositions and methods of the present invention provide relative to any method which seeks to inhibit proliferation through the use of IFN alone.

The administration of IFN and any dsRNA, i . e., perfectly base-paired or mismatched, "in combination" includes embodiments in which both agents are administered together as a therapeutic mixture, and also when both agents are administered separate but simultaneously, e.g., as through separate intravenous lines into the same individual. Administration "in combination" further includes the separate administration of these drugs, in which one of the drugs is given first and followed shortly thereafter by the second. All three of these embodiments posses inherent advantages. Separate but simultaneous administration, for example, allows independent control of each agent, and thereby provides for therapeutic optimization on an individual patient basis. Separate administration allows for IFN alone to establish an effect on cells, however marginal, which effect is then amplified by the use of the dsRNA. Preparation of IFN and dsRNA as a mixture, of course, provides for instant access to this therapeutic composition in cases where optimum levels have previously been established. The preceding comments apply to all cases presented in this specification, including the claims, where one therapeutic agent is administered "in combination" with another.

Similarly, the therapeutic agents and compositions of this invention can be administered via any of the bodily routes customarily used and known in the medical arts. Intravenous administration has been mentioned, but other routes including intramuscular, intrathecal, intracranial, and intraperitoneal are also well within the scope of this disclosure.

Lymphokines include the interferons (alpha, beta, gamma), preferably interferon alpha, the interleukins, specifically interleukins (1, 2 or 3) and recombinant interleukin-2 (rIL-2), and tumor necrosis factor (TNF). Also included are lymphokine activated killer cells (LAK) formed in animals in response to exposure to a lymphokine.

When interferon (alpha) is used as the lymphokine, an amount of from 0.01 to 100,00 IRU per milliliter of the patient's body fluid is provided. When IL-2, preferably rIL-2 is the lymphokine, the amount administered lies within a range of about $10^2$ IL-2 units per kg of the patient's body weight up to a value approaching unacceptable levels of toxicity in that patient, which may be as high as $10^6$ IL-2 units. However, in the range of from about $10^3$ to about $10^4$ IL-2 per kg of body weight.

When both agents, the dsRNA and the lymphokine, are administered as previously described they may be administered as a mixture, administered separately but simultaneously, or sequentially.

When the lymphokine and any dsRNA are administered in combination, the dsRNA may be administered in an amount which will result in a level from 1-1,000 $\mu$g dsRNA/ml in bodily fluid, i.e., that solution of serum, salts, vitamins, etc., which circulates within the organism and bathes the tissues. For example, administration of a composition containing 10 mg of dsRNA to an individual weighing 160 lb. would result in a dsRNA level of approximately 1$\mu$g/ml. Similarly, the amount of IFN present when administering a dsRNA-IFN combination is that amount which would result in a level of 1-100,000 IRU/ml in bodily fluid. The manner of administration of the combination may be as previously described, i.e., as a mixture, administration separately but simultaneously, or sequentially. The point here is that the administration be functionally synergistic, whatever physical embodiment it takes.

By "mismatched dsRNA" are meant those in which hydrogen bonding (base stacking) between the counterpart strands is relatively intact, i.e., is interrupted on average less than one base pair in every 29 consecutive base residues. The term "mismatched dsRNA" should be understood accordingly. The dsRNA may be a complex of a polyinosinate and a polycytidylate containing a proportion of uracil bases or guanidine bases, e.g., from 1 in 5 to 1 in 30 such bases (poly I•poly ($C_4$-$C_{29}$ x > U or G). Alternatively, appropriate oligonucleotides (small nucleotide fragments) may be complexed with appropriate complementary polynucleotides or oligunucleotides under certain circumstances.

The dsRNA may be poly I•poly C,U in which the ratio of C to U is about 13 to 1 and the sedimentation coefficients of poly I and poly C,U are both less than 9 and within 2 units of each other, and both preferably about 6.5 to 7.5.

The dsRNA may be of the general formula $rI_n$•$r(C_{11-14},U)_n$ and specifically, $rI_n$•$r(C_{12},U)_n$. Other suitable examples of dsRNA are discussed below.

The mismatched dsRNAs preferred for use in the present invention are based on copolynucleotides selected from poly ($C_n$,U) and poly ($C_n$,G) in which n is an integer having a value of from 4 to 29 and are mismatched analogs of complexes of polyribocytidylate ($rC_n$), e.g., by including 2'-0-methyl ribosyl residues. These mismatched analogs of $rI_n$•$rC_n$, preferred ones of which are of the genera formula $rI_n$•$r$-($C_{12}$,U)$_n$, are described by Carter and Ts'o in U.S. Patents 4,130,641 and 4,024,222. The dsRNA's described therein generally are suitable for use according to the present invention.

Specific examples of mismatched dsRNA for use in the invention include: -

poly (I)•poly ($C_4$,U)
poly (1)•poly ($C_7$,U)
poly (I)•poly ($C_{13}$,U)
poly (I)•poly ($C_{22}$,U)
poly (I)•poly ($C_{20}$,G)
poly (I)•poly ($C_{29}$,G) and
poly (I)•poly ($C_p$) 23 G>P

The amount of mismatched dsRNA administered is preferably sufficient to achieve a peak blood concentration of from 0.01 micrograms per milliliter of dsRNA up to 1000 micrograms per milliliter of dsRNA in the systemic blood circulation immediately following administration distal from the point of infusion.

As previously stated, a preferred form of the present invention occurs when the dsRNA employed to inhibit proliferation of neoplastic cells is a mismatched dsRNA such as $rI_n$•$rC_n$($C_{11-14}$,U)$_n$. We have found that mismatched dsRNAs cause milder pharmacologic effects relative to perfectly base paired dsRNAs.

The activities of other IFN types besides human fibroblast IFN were also examined, including the natural varieties of human leukocyte IFN and IFNs produced in bacteria by use of recombinant DNA technology (rIFN). In all cases, the IFNs demonstrated less effect on human tumor cell growth when injected alone than when injected together with or followed by dsRNA, whether matched ($rI_n$•$rC_n$) or mismatched $rI_n$•$r(C_{11-14}$,U)$_n$). The maximum reduction in human tumor cell growth achieved by any IFN administered alone was approximately 33% across a broad range of injected IFN concentrations ($10^5$ to $3 \times 10^6$ IRU daily). In contrast, animals treated with as little as $10^5$ IRU daily of IFN in combination with a dsRNA exhibited a 65% or greater reduction in human tumor cell growth. Accordingly, dsRNA used in conjunction with any single form of IFN results in a quantitatively superior effect. Moreover, human tumors generally require an individualized treatment regimen to generate the optimum therapeutic effect. Further efforts to individualize the treatment schedule are expected to result in a 10-fold or greater therapeutic effectiveness wherever a mismatched dsRNA is used in conjunction with IFN.

The data clearly demonstrate that dsRNA will amplify the therapeutic effectiveness of all forms of IFN, including natural, synthetic and the hybrid forms, such as those derived in part from alpha IFNs and in part from beta or gamma IFNs, and all are within the scope of this invention. Accordingly, the combination of dsRNA, especially a mismatched dsRNA, notably $rI_n$•$r(C_{11-14}$,U), constitutes a potent formulation against cancer far exceeding the capability of IFN alone. Moreover, the human tumor material studied is known to

7

contain various types of viral genetic information which affirmatively contribute to the malignant process and/or the pathological processes in human tissue, causing tissue disease and destruction. The data therefore importantly demonstrate that a dsRNA-IFN combination is not only an effective anti-cancer agent, but also acts against viral components and diseases as well. Indeed, the therapeutic compositions of this invention will act against any disease in which IFN treatment may be indicated, including viral diseases manifested by either acute, subacute, latent, or chronic phase, and also including those dysimmune human diseases in which viral activity serves to initiate the human disease pathology but may also disappear as the human "autoimmune" disease becomes self-perpetuating.

It should be emphasized that the role of any dsRNA in synergistic enhancement of any combined dsRNA-IFN treatment is not as an IFN inducer, else no synergism would be observed. Rather, the mismatched dsRNA is a uniquely contributing agent which supplements and complements the action of IFN, thereby providing a degree of flexibility and effectiveness which could not be attained by either dsRNA or IFN alone in the treatment of human viral diseases and cancer.

I have observed low levels of NK cell activity in some members of a study group including patients with lung cancer and individuals with a high family history of cancer. These observations are to be detailed below. I have tested whether low NK cell activity in patients with high family history of cancer could be re-established to normal levels through the addition of various types of interferon and/or mismatched dsRNA. Recent reports demonstrate differences in the efficiency of NK cell augmentation (cells derived from normal individuals) among both natural types (Brit. J. Maem., 50, 85, 1982) and cloned subtypes (Can. Res., 42 1312, 1982) of interferon. I observed that mismatched dsRNA has the most pronounced ability to re-establish normal levels of NK cell augmentation in individuals at risk to the development of cancer.

The invention is further explained in the following examples in which all parts and percentages are by weight. The dsRNA (mismatched) used in the examples that follow was $rI_n \cdot r(C_{11-14},U)_n$, sometimes noted as $rI_n \cdot (C_{12},U)_n$.

## A. dsRNA's Novel Mechanism of Growth Modulation

The novel mechanism of growth modulation exhibited by mismatched dsRNAs is not shared by prototype lymphokines. This study demonstrates dsRNA's broad base utility in growth modulation of aberrant human cells and by a mechanism other than the induction of INF. Specifically, the dsRNA induction of a cyclic adenosine monophosphate (cAMP) was found in cells known to be insensitive to interferon-alpha. These studies were conducted with two cAMP kinase inhibitors designated H-7 and HA 1004 (known metabolic inhibitors of protein kinase C and cAMP kinase) to assist in evaluating the novel antigrowth modulating activity of mismatched dsRNA in the face of resistance to single modality lymphokine therapy using interferon-alpha as a prototype lymphokine.

To demonstrate mismatched dsRNA does not function by inducing the production of interferon in the IFN-insensitive cells, human glioma (brain tumor) cells (A 1235) were treated with the dsRNA for different periods of time ranging from 0 to 8 hours. The dsRNA was removed and fresh medium added for 24 hours, then the interferon titers (IRU/ml) were determined by cytopathic effect assay (Finter, N.G., J. Gen Virol. 5:419-427, 1969). The results are reported in the following table in which the lower limit of detection was 5 IRU/ml.

TABLE 1

| Interferon Induction in dsRNA-Treated Human Glioma Cells | | | | | |
|---|---|---|---|---|---|
| Indicator Cell | Length of Treatment (hr) | | | | |
| | 0 | 2 | 4 | 6 | 8 |
| HFF | <5 | <5 | <5 | <5 | <5 |
| WISH | <5 | <5 | <5 | <5 | <5 |

From this it was concluded that the dsRNA does not induce interferon in these IFN-insensitive cells.

Next, similar cells were subjected to a confirmatory test to determine if interferons play any role in the antiproliferative effects caused by the dsRNA in the cells in question. This was done by the use of antibodies to interferon which, if present, would bind to the antibody and reduce the antiproliferative dsRNA-induced effect. Three different antibodies to interferons (alpha, beta, gamma) at 240 neutralizing units/ml and one blank or control were used. Cell growth inhibition assays were performed as described by Hubbell et al, Cancer Res. 44:3252-3257 (1984). Percentage of control growth for the antibody was calculated as follows:

$$\frac{\text{treated cells (24 hr) - control cells (0 hr)}}{\text{control cells (24 hr) - control cells (0 hr)}} \times 100$$

The results are reported in the following table:

## TABLE 2

### Antibodies to IFNs Do Not Inhibit dsRNA-Induced Antiproliferation in Human Glioma Cells

| Treatment | Conc. | Antibody | | | |
|---|---|---|---|---|---|
| | | 0 | anti-αIFN | anti-βIFN | anti-γIFN |
| A1235 | | | | | |
| 0 | | | 91.8±5.9 | 90.8±7.4 | 87.8±3.1 |
| Ampligen | 200 g/ml | 59.9±4.6 | 55.7±2.1 | 50.8±1.7 | 56.5±2.0 |
| α-IFN | 100 IRU/ml | 41.4±4.6 | 91.8±3.0 | ND | ND |
| β-IFN | 100 IRU/ml | 40.9±2.3 | ND | 84.9±3.9 | ND |
| RT4 | | | | | |
| 0 | | | ND | ND | 108.9±5.6 |
| γ-IFN | 25 IRU/ml | 70.2±3.8 | ND | ND | 199.1±7.1 |

Results reported are percent of control growth at 24 hours. ND = not done. This data again confirms that the ds RNA does not induce IFN in the cells studied.

The dsRNA was found to induce cyclic AMP in human glioma cells which was directly attributable to the dsRNA and not from induction of any INF in these cells. Three known metabolic inhibitors, H-7, HA 1004 and pertussis toxin, inhibit or antagonize the dsRNA antitumor effect as reported in the following tables. Percent cell growth was again assessed, as in Table 2, above, the object to obtain a low percent of control growth. The mismatched dsRNA alone works to reduce the percent control growth; however, the presence of the metabolic inhibitor increases cell growth.

Antagonism of the mismatched dsRNA antitumor effect by H-7 and AN 1004 metabolic inhibitors on human glioma cells is shown in Table 3, below, the values again reported as percent control growth for the dsRNA in concentrations of 0 (blank, or control), 25, and 200μg/ml.

## TABLE 3

| | dsRNA (µg/ml) | | |
|---|---|---|---|
| | 0 | 25 | 200 |
| **H-7 (µM)** | | | |
| 0 | | 66.7±12.6 | 48.2± 5.4 |
| 5 | 103.3±13.6 | 108.3±15.5 | 75.2± 4.5 |
| 25 | 87.5± 6.7 | 89.7±11.0 | 72.8± 7.9 |
| **HA1004 (µM)** | | | |
| 5 | 112.7±16.2 | 113.4± 3.2 | 94.1±13.4 |
| 25 | 113.2±14.3 | 117.2±16.7 | 101.7± 6.8 |

Similarly, pretreatment of the same human glioma cells with pertussis toxin inhibits dsRNA-induced antiproliferation, as shown below. In Table 4, percent control growth is reported at 24 hours in cells pretreated for 4 hours with pertussis toxin prior to addition of the dsRNA. This demonstrates that H-7 and HA 1004, two recognized metabolic inhibitors, have minimal effect on growth inhibition in interferon-alpha treated cells, while pertussis toxin appears to have a biphasic effect --no change in interferon-alpha-induced growth inhibition at higher doses and enhancement of the antiproliferative effect at lower dose.

TABLE 4

| Treatment | dsRNA (µg/ml) | | |
|---|---|---|---|
| | 0 | 25 | 200 |
| 0 | | 88.4±2.1[a] | 71.7±5.7 |
| 3 µg/ml | 98.7±7.1 | 102.5±7.3 | 99.9±6.3 |
| 1 µg/ml | 94.0±5.2 | 93.8±7.1 | 93.2±5.1 |
| 0.3 µg/ml | 99.4±7.2 | 95.6±4.1 | 102.7±5.7 |

On the other hand, IFN-alpha does not increase intracellular cAMP levels in the same cells throughout a 24 hour period, as shown in the following table. In this procedure, the human glioma cells (A 1235) were exposed to zero (control), 250 and 1000 IRU/ml of IFN-alpha for times ranging from the start of the experiment to 24 hours.

TABLE 5

| Length of Treatment | IFN-α (IRU/ml) | | |
|---|---|---|---|
| | 0 | 250 | 1000 |
| 0 | <0.02 | | |
| 1 min | | <0.02 | <0.02 |
| 5 min | | <0.02 | <0.02 |
| 10 min | | <0.02 | <0.02 |
| 30 min | | <0.02 | <0.02 |
| 1 hr | | <0.02 | <0.02 |
| 2 hr | | <0.02 | <0.02 |
| 4 hr | | <0.02 | <0.02 |
| 8 hr | | <0.02 | <0.02 |
| 24 hr | | <0.02 | <0.02 |

In this table, the values are reported as pica moles cAMP/$\mu$g protein; the limit of detection was 0.02 p moles cAMP/$\mu$g protein.

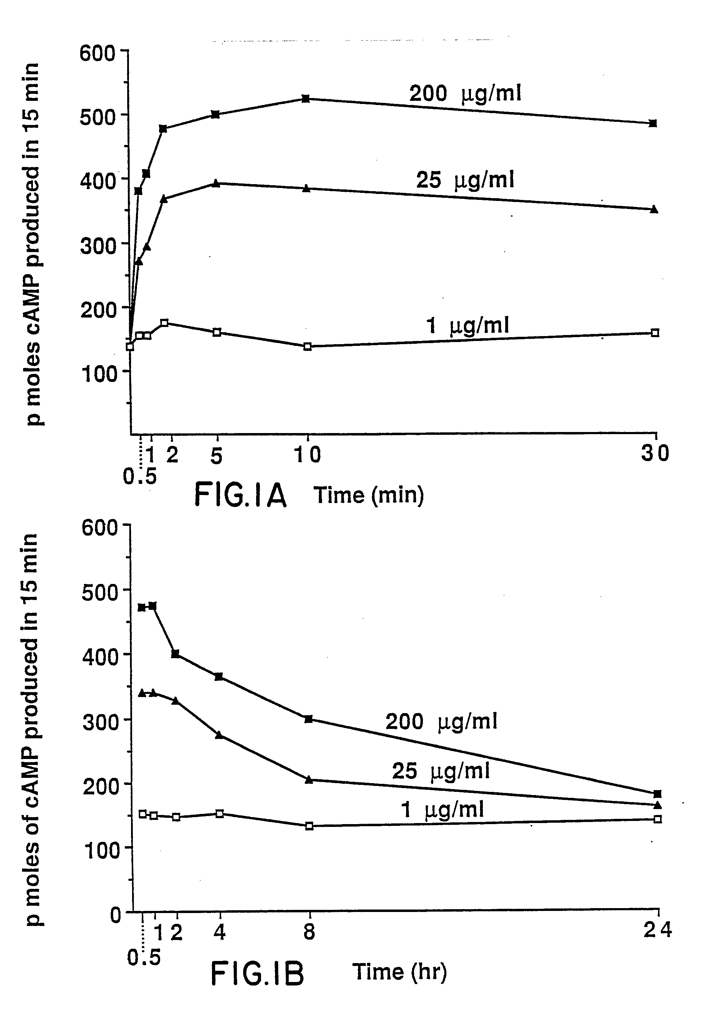

Intracellular cAMP levels were next measured indirectly in dsRNA-treated human glioma cells (A 1235) by measuring the adenylate catalyse activity (p moles of cAMP produced in 15 minutes) immediately following antiproliferative doses of the dsRNA (25 and 200 $\mu$g/ml), in 30 minutes as shown in Figure 1A, and following 24 hours in Figure 1B. In this procedure, the cells were treated with mismatched dsRNA, 25 (solid triangle) or 200 (solid square) $\mu$g/ml of mismatched dsRNA. At the appropriate time points (Fig. 1A: 0.5-30 min and Fig. 1B: 0.5-25 hr) the medium was removed and cells were quick frozen on dry ice. Adenylate cyclase activity in cell sonicates was assayed by the method of Young, et al, Molec. Endocrinol. 1:884-888, 1987. Cell counts were done at 24 hours to verify antiproliferative activity.

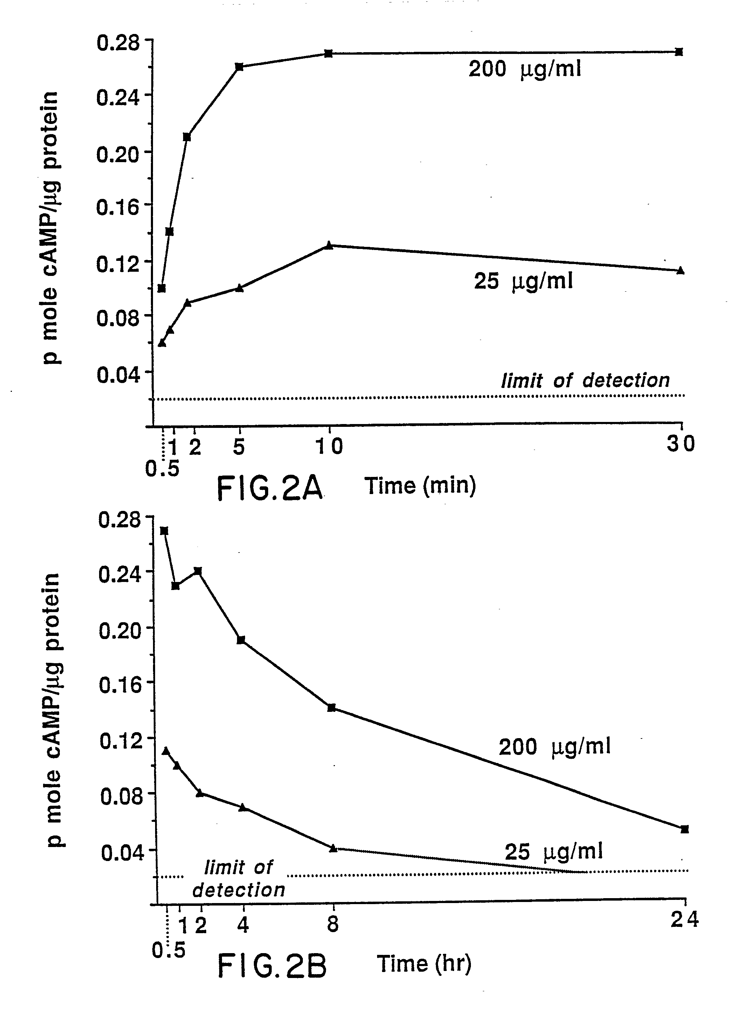

Induction of cAMP in dsRNA-treated human glioma cells (A 1235) was measured directly as pica moles of cAMP per microgram of protein and the results shown in Figure 2A over 30 minutes and Figure 2B over 24 hours. In this procedure, the A 1235 cells were treated with 25 (triangle) or 200 (solid square) $\mu$g/ml of the mismatched dsRNA. At the appropriate time points, medium was removed and the intracellular cAMP was solubilized in 0.1N HCl. cAMP levels were determined using a RIA method as described by Reisine et al, J. Cell. Biol 102:1630-1637, 1986. cAMP levels in control cells and cells treated with 1$\mu$g/ml of the dsRNA were below the limit of detection. Cell counts were done at 24 hr to verify antiproliferative activity.

In these four graphs, note the dramatic increase in cAMP after only 30 seconds exposure to the dsRNA (Figs 1A and 2A) and the continued levels over 30 minutes. The measurements over 24 hours as in Figs. 1B and 2B show the effect of the dsRNA over time.

These studies confirm the increase of cAMP levels was directly attributable to the dsRNA that since the dsRNA does not induce IFN in the cells used. Antiproliferative doses of the dsRNA induce adenylate cyclase activity within 30 seconds after the initiation of treatment. Similarly, intracellular cAMP levels were increased in an antiproliferative dose-dependent manner within 30 seconds. Pretreatment of the cells with pertussis toxin, also an inhibitor of intracellular cAMP followed by dsRNA treatment inhibits dsRNA induced growth inhibitor. In contrast, antiproliferative doses of natural human IFN-alpha do not increase intracellular cAMP levels throughout a 24 hour treatment period. In addition, H-7 and HA 1004, both known metabolic inhibitors, have minimal effect on growth inhibition in IFN-alpha treated cells, while pertussis toxin appears to have a biphasic effect, as noted above.

In studies of the cAMP system in IFN-induced antiproliferation (Panniers et al, J. Cell Sci., 48:259, 1981; Banerjee et al, Virology 129:230, 1983; Ebsworth et al, J. Cell Physiol. 120:146, 1984), these authors found that cAMP was not associated with degree of antiproliferation state. To my knowledge, there have been no studies, prior to those reported herein, on dsRNAs in the same system. These studies support the conclusions that (a) dsRNAs and the lymphokines, of which IFN is a prototype, have different mechanisms of action, and (b) dsRNA-induced antiproliferation is associated with the cAMP system (while the lymphokines are not).

In that cAMP is ubiquitous in nature, i.e., most if not all cells possess the genetic information needed to synthesize a cAMP under the appropriate stimuli, these experiments indicate a broad base of the mismatched dsRNA utility in the growth modulation of aberrant human cells, these cells being relatively or completely resistant to IFN or other lymphokines given alone. These results further suggest that mismatched dsRNA acts by two sequential mechanisms -- the first mediated by cAMP kinase, followed by modulation of protein kinase C.

## B. Protein Synthesis

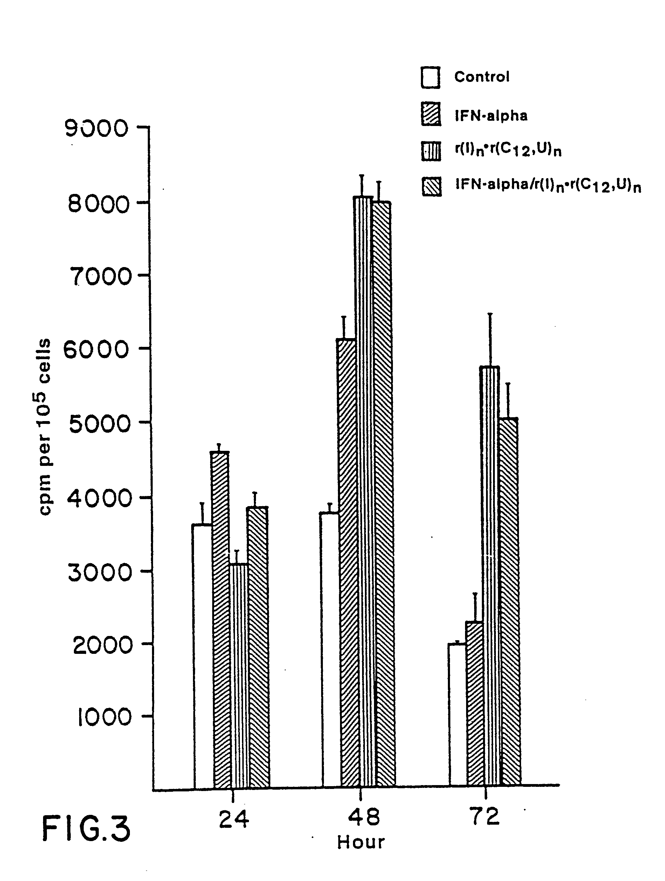

Intracellular protein changes in dsRNA-treated cells reflect progressive differentiation and loss of malignant cell phenotype, a non-lymphokine property. Cell normalization following dsRNA treatment was studied in the manner of Soslau et al, Biochemical and Biophysical Research Communications, 119:941-948, 1984. In this procedure, human brain tumor (A 1235) cells treated with antiproliferative doses of mismatched dsRNA (200 $\mu$m/ml) or natural human IFN alpha (100 $\mu$m/ml) showed significantly different patterns of protein synthesis over a 72 hour period. Protein systhesis is an important parameter as it indicates that treated abnormal cells are normalizing and it indicates the length or duration of the therapeutic effect. The results of this study are represented in the bar graph of Figure 3. Protein synthesis was assessed by measuring the counts per minute from radiolabeled A 1235 cells for a preset number of cells. These cells were untreated (control, open bar), treated with IFN-alpha only at a concentration of 100 $\mu$m/ml (angle lines from upper right to lower left), the dsRNA (verticle lines) at 200 $\mu$m/ml, and both IFN-alpha and dsRNA together in the quantities indicated (angle lines from upper left to lower right). Measurements were made following 24, 48 and 72 hours. The single line above each bar represents one standard deviation unit.

Although at 24 hours nominal changes were seen in either agent compared to controls, by 48 hours both dsRNA and IFN showed significant increases in protein synthesis with dsRNA-stimulated protein synthesis being significantly higher than that of the IFN-treated cells. Surprising was the 3-fold increase in protein synthesis in the dsRNA-treated cells compared to controls and IFN-treated cells, in which protein synthesis dropped to control levels. The continued increase in protein synthesis in dsRNA-treated cells apparently reflects significant changes in the types of specific proteins produced as a result of dsRNA-induced cellular changes to a more differentiated or normalized cellular phenotype.

Studies of protein phosphorylation in human bladder carcinoma cells treated with interferon-beta showed significant changes in the phosphorylation of a number of proteins compared to untreated cells (Soslau et al, Biochem. Biophy. Acta, Vol. 119, 1984, p. 941). In contrast, my present studies (polyacrylamide gels not shown) with $rI_n \cdot r(C_{11-14}, U)_n$ demonstrate entirely different changes in phosphorylation patterns when compared with the interferon-treated cells. These data further strengthen the molecular differences between dsRNAs and lymphokines in terms of mechanisms of tumor cell modulation, reversion to more normal cellular phenotypes, and fundamental differences in pathways utilized in modulation of immunodifferentiation and immuno-competence.

To one skilled in oncologic/immunologic art, in the practice of the basic invention, here disclosed in a variety of manners, one will select a specific protein characteristic of the malignant process for a given cell of interest, or a characteristic of the recovery of the malignant cell of interest, and monitor changes in the selected protein or group of proteins in light of the invention here described. Factors that vary from subject to subject can effect the net requirement for mismatched dsRNA over a period of time while not undermining the basic utility of the invention. Note - the presence of unusually high levels of nucleases or phosphodiesterases could change or cause the clinician to modify the concentration of dsRNA therapy required as opposed to those presented from the examples and experiences here cited. Variation from patient to patient is to be expected, as with many aspects of clinical medicine.

Protein synthesis studies enable one to more fully track the underlying mechanisms; determine the kinetics of onset of drug action; the magnitude of drug action, thus assess the number of cells normalizing and the duration of drug action, hence the duration of therapeutic effect, all in a manner not foreshadowed by applicant's extensively reported prior efforts in this field.

## C. Intrinsic dsRNA Required in Tumor Cells for IFN Antigrowth Activity

I have determined that interferon antigrowth activity in tumor cells occurs only rarely and primarily when the IFN-sensitive tumor cells have pre-existing intrinsic dsRNA in a sufficient amount resident in the cells. Antigrowth activity in cells lacking any or sufficient intrinsic dsRNA may be coaxed into antigrowth activity by creating dsRNA (administering it) to create conditions of dsRNA sufficiency.

Interferons are known to induce the double-stranded RNA-dependent enzyme $2',5'$ oligo A synthetase which has been implicated in cellular growth inhibition. My discoveries indicate that the double-stranded RNAs work essentially as co-factors for the activation of the enzyme, a distinct step from the interferon induction of this enzyme. Historically, these two actions have been erroneously grouped together resulting in a dramatic diminution in the potential for therapeutic benefits of either agent, alone or in combination. I now show that dsRNAs occur naturally in the peripheral blood mononuclear cells (PBMCs) of certain individuals, namely those with hairy cell leukemia, although they are not found in the PBMCs of normal individuals. Indeed, their presence in hairy cell leukemia patients apparently now explains for the first time the known interferon effectiveness in such cases of interferon given alone and provides a critical additional dichotomy between IFN and dsRNA which can be exploited therapeutically. Namely, interferon clinical effectiveness is due to enzyme induction ($2',5'$ oligo A synthetase) in the presence of pre-existing intracellular natural dsRNA, whereas exogenous dsRNA works largely by activation of preexisting enzymes in this particular pathway of growth control.

To validate my novel hypothesis, I isolated nuclear RNAs from control and rIFN-$\alpha$A treated hairy cell leukemia (HeLa) cells, fractionated on sucrose gradients and tested for their ability to activate purified high molecular weight $2',5'$A synthetase. None of the fractions of the untreated HeLa cell RNAs could activate the synthetase. However, the heterogeneous nuclear RNA (HnRNA) fraction of the IFN treated cells activated the synthetase in a dose-dependent manner. Heat denaturation of the HnRNA abolished this activation. High pressure liquid chromotography (HPLC) analysis of the enzymatic products indicated that biologically active $2',5'$A trimers and tetramers were formed. I also fractionated mononuclear cell nuclear RNA from hairy cell leukemia patients, who are extremely sensitive to IFN therapy and demonstrated high levels of $2',5'$A synthetase ctivating dsRNAs in the HnRNA fraction. HPLC analysis showed the formation of biologically active $2',5'$A trimer, tetramer, pentamer and hexamer (relative ratios of approximately 53:15:4:1). Normal mononuclear cell nuclear RNAs yielded no activation. These results indicate that natural, nuclear RNAs exist which when present in the relevant cells, neoplastic or otherwise, can participate in growth regulation by IFN. Indeed, this is a hitherto undetected critical necessity for an "interferon response". This then allowed me to deduce that deficiencies in these dsRNAs can lead to IFN ineffectiveness, which can, however, be overcome by exogenous dsRNA, a point which I was then able to test and confirm in a clinical setting as I describe elsewhere in this application.

To be successful, IFN activity requires the presence of rare dsRNA resident in the cell in which antigrowth activity is desired. This appears to be required for the tumor cell to be receptive/sensitive to IFN therapy. Some reviewers wrongly group the IFNs and dsRNAs together. From these and related studies it appears that dsRNAs are cofactors which activate the enzyme $2',5'$ oligo A synthetase. Tumor cells insensitive to IFNs, when supplied with exogenous dsRNA, become susceptible to successful therapy with dsRNA. In cells responding to IFN treatment, the intracellular dsRNA or dsRNAs are believed to have a three-dimensional structure, highly similar to the mismatched dsRNAs, notably $rI_n \cdot r(C_{11-14},U)_n$ of this invention on the basis of their similar biological, catalytic and lack of toxicity properties, enhanced immune function as compared with cytotoxic function as well as other data such as cAMP and tumor cell parameters.

Cells non-responsive to lymphokines in general, and IFNs in particular are rendered responsive by first supplying an effective quantity of exogenous dsRNA to induce dsRNA synthesis of the desired intracellular dsRNA which participates in cell growth regulation in association with an interferon, and then supplying the now therapeutic IFN while optionally continuing to supply the dsRNA, as may be required.

## D. Human Kidney Tumor Xenograft Responses

The response of human kidney xenografts to IFN and dsRNA are fundamentally different in terms of long term survivors and magnitude of effect, as explained in the following investigation.

I have utilized a number of novel animal systems utilizing human tumor xenografts in athymic mice to show an unexpected and differential response to interferons and dsRNA. For example. human renal cell carcinoma demonstrated progressive tumor growth and no increase in survival during interferon-alpha treatment, whereas 1 observed significant decreases in tumor size and dramatic increases in survival with dsRNA treatment. Indeed, after death at 2-4 years of age from natural causes, 95% of the dsRNA treated mice had no histologically present tumor at autopsy. This was most unexpected since lymphokine (e.g., IFN) induced remissions from cancer are notoriously short-lived and there are few, if any, examples of tumor-free subjects even after several months. Thus the observation of a 95% cure rate by histological criteria and survival data are most unexpected. In addition, splenic natural killer (NK) cell activity was augmented in dsRNA-treated animals while no increases in NK activity were seen in interferon treated mice.

Similar differences in growth inhibition were seen between interferon-gamma and dsRNA in two different xenograft models, and I have also observed pronounced differences in the human bladder carcinoma RT4 and the human brain tumor glioma A 1235 as reported above. In all cases, statistically significant inhibition of tumor growth was seen in dsRNA-treted animals while minimal growth inhibition was seen with interferon-gamma alone. Significant differences were also seen in splenic NK activity, with augmentation of NK activity by dsRNA, but no increase in NK activity by interferon-gamma.

Similar effects were observed in terms of immuno augmentation; results with brain tumors are shown in accompanying Table 6. Dosages were 20,000 IRU/interferon daily vs. $rI_n \cdot r(C_{11-14},U)_n$ given 2 or 3 times per week (200-500 $\mu$g per dose). Careful study of blood samples (obtained serially over time from tail vein) showed that dsRNA, of the chemical class described elsewhere in the application, could be administered indefinitely without any untoward effect on renal, bone marrow and immune function, which have been historically organs expressing toxicity.

In Table 6 that follows, immune cell activity in spleen cells of mice treated with IFN-gamma and/or dsRNA is reported. The study mixes effector cells (immune cells obtained by surgery from the spleen of animals bearing human tumor xenografts) to target cells, (in this case, human brain tumor or cells) in the indicated ratio. The effector cell is an immune cell of the immune system and the target cell is a cell of the specific cancer of interest. Results are reported as percent cytotoxicity (cell death) with the object to achieve a higher number (percent) as a result of the treatment (IFN, dsRNA or both). Higher percent toxicity than the control indicate improved tumor cell eradication.

TABLE 6

| Effector Cell Treatment | Control |
|---|---|
| Control | |
| 200:1 | 16.3 |
| 100:1 | 14.2 |
| 50:1 | 7.5 |
| IFN gamma | |
| 200:1 | 6.4 |
| 100:1 | 2.9 |
| 50:1 | 2.8 |
| dsRNA | |
| 200:1 | 32.3 |
| 100:1 | 22.8 |
| 50:1 | 13.2 |
| IFN gamma + dsRNA | |
| 200:1 | 28.7 |
| 100:1 | 22.8 |
| 50:1 | 17.2 |

Curiously, in this test IFN-gamma killed fewer cells than in the control group (no treatment), while dsRNA alone provided best results with the combination treatment close behind.

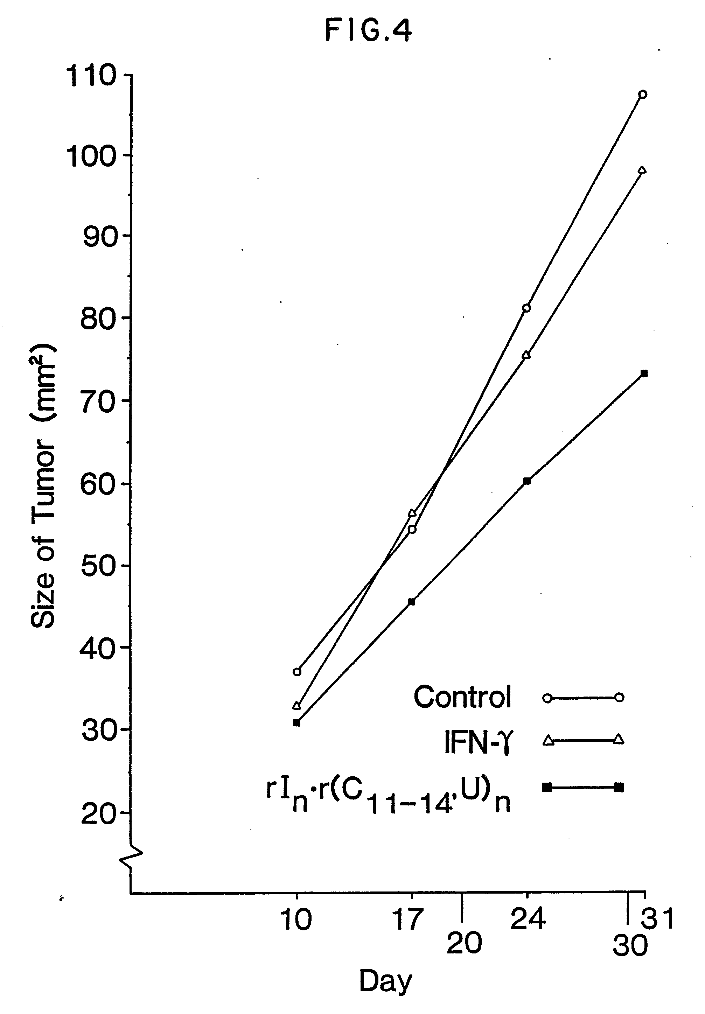

The effectiveness of dsRNA, specifically $rI_n \cdot r(C_{11-14},U)_n$, against human brain tumors engrafted in athymic mice as compared with IFN-gamma is illustrated in Figure 4 of the drawings. This is a graph comparing the two-dimensional size of the tumor, calculated in length times width, in mm$^2$. The line connecting the open circles is the control group (no therapy); the line connecting the open triangles is IFN-gamma alone (P>0.5); the line connecting the solid squares is the dsRNA $rI_n \cdot r(C_{11-14},U)_n$ (p>0.50). Tumors are measured from the 10th to the 31st day following initiation of the experiment. The dsRNA was effective in reducing the tumor size as compared with IFN-gamma which was only slightly more effective than the control.

E. Clinical Examples of Lymphokine-Resistant Tumors Which Respond to dsRNA

Alternative methodology was developed, different from an animal model, to evaluate tumors susceptible or resistant to IFN therapy, and to identify those instances in which potential clinical synergy is likely for combination IFN and dsRNA therapy. One such technique is the human tumor cologenic assay technique deveolped by Hamburber and Salmon (Primary Bioassay of Human Tumor Stem Cells, Science 197:461-463, 1977), which can reliably predict clinical response or resistance to anticancer drug in individual patients with great accuracy on the cellular level at 90-95% reliability for inactive drugs and 75-80% for active drugs.

The colonogenic assay is conducted according to Hamburger and Salmon's procedures as follows. Fresh tumor cells or leukemic cells are obtained from the patient and prepared into a single cell suspension. A predetermined concentration of the candidate therapeutic agent, calculated from literature and other dosage guidelines, is applied to the agar or methylcellulose plate or to cells after plating. The cells are washed and plated onto agar or methylcellulose and incubated in petri dishes at 37° C to permit cell colony growth. Following a predetermined period of cell growth, the colonies are inspected visually with a microscope and the number of colonies in each petri dish are counted. The biology and cellular composition of the colonies and then studied in greater detail, including cell morphology, karyology, self-renewal, immunology, and the effects of the cells in athymic (nude) mice. The cells are analyzed using monoclonal antibodies and nucleic acid probes. Cologenic assays represent a reliable approach in in vitro tumor cell chemosensitivity testing.

Colonogenic assay tests were conducted on several tumor specimens in the inventor's laboratory. Contrary to expected lack of antitumor activity reported in various cancer treatment symposia (Compilation of Phase II Results with Single Antineoplastic Agents, U.S. Department of Health and Human Services, Public Health Service, National Institutes of Health, Volume 4, 1985), reporting about 100 individuals who received poly I•poly C without any complete or partial responses, the inventor observed a 42% predicted response rate using a mismatched dsRNA in a wide variety of human solid tumors historically insensitive to polynucleotide therapy.

For purposes of preliminary clinical evaluation, tumors regarded as sensitive showed greater than 60% cell decrease in the number of tumor cell clones.

In a vast majority of instances, concurrent tests, both in the laboratory and in the clinic, were conducted on individuals to determine their tumor sensitivity to various generic classes of IFN and/or various types of interleukins. Specifically, individuals who participated in dsRNA studies had undergone previous unsuccessful clinical treatments with the interferons or the interleukins at doses believed to be effications based upon published medical data. In all instances the methodology with the specific lymphokine was unsuccessful in preventing further tumor growth, and in a majority of instances immune cell profiles were unchanged or in many instances actually decreased (worsened) over the values seen prior to infusing the specific lymphokine alone.

The clinical laboratory arm of the present invention established invention established at least three unexpected results:
• the mismatched dsRNA of choice works when poly I•poly C does not;
• the mismatched dsRNA of choice is effective when IFN alone is ineffective;
• synergism occurs when both the mismatched dsRNA of choice and a lymphokine are employed.

The relative sensitivity of various histologic types of human tumors to the mismatched dsRNA has been studied (see Table 7 below). Experience indicates more than 42% of a large assortment of various tumors, especially solid tumors, although resistant to exposure to lymphokines alone are exquisitely sensitive to the antiproliferative effects of the specific molecular configuration of dsRNAs.

TABLE 7

| Histologic Type | Number Sensitive | Number Resistant | Percent Sensitive |
|---|---|---|---|
| Endometrial carcinoma | 2 | 0 | 100 |
| Glioblastoma | 4 | 1 | 80 |
| Lung carcinoma | 3 | 2 | 60 |
| Renal cell carcinoma | 33 | 30 | 52 |
| Carcinoid | 3 | 3 | 50 |
| Prostatic carcinoma | 1 | 1 | 50 |
| Ovarian carcinoma | 4 | 7 | 36 |
| Breast carcinoma | 5 | 9 | 36 |
| Melanoma | 5 | 9 | 36 |
| Colo-rectal carcinoma | 1 | 7 | 13 |
| Sarcoma | 0 | 10 | 0 |
| Esophageal carcinoma | 0 | 2 | 0 |
| Mesothelioma | 0 | 1 | 0 |
| Pancreatic carcinoma | 0 | 1 | 0 |
| Gastric carcinoma | 0 | 1 | 0 |
| Medulloblastoma | 0 | 1 | 0 |
| Total (146) | 61 | 85 | 42% |

This observation is unexpected for at least two reasons -- the historical bioinertness of dsRNA in general and the clinical lack of utility in lymphokines.

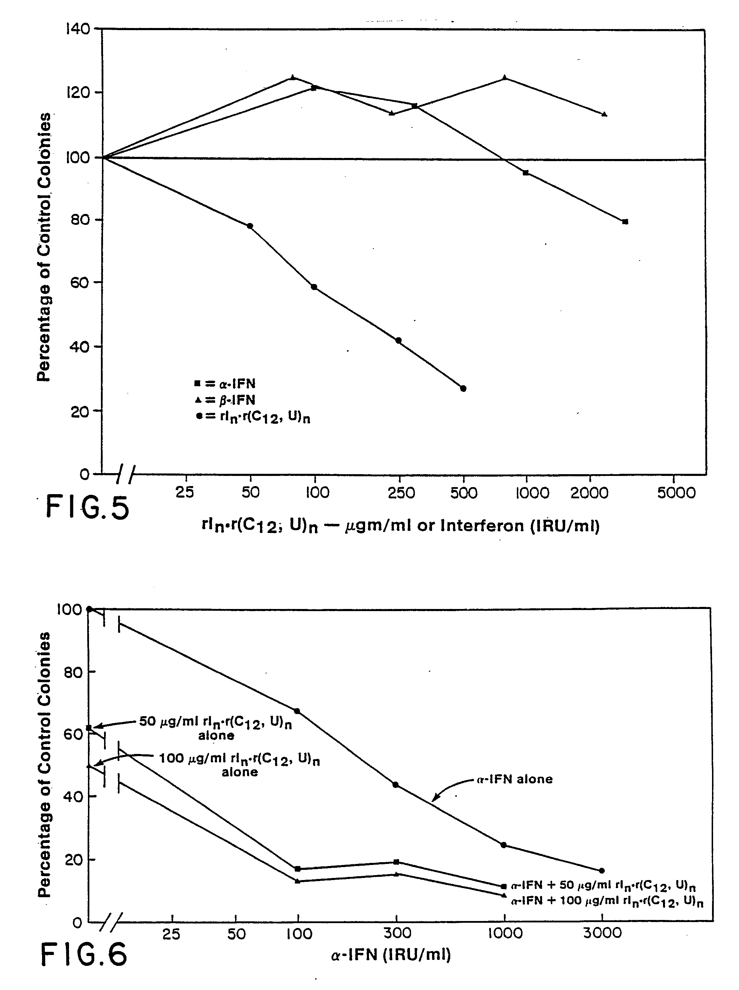

The effects of IFN-alpha, IFN-beta and the mismatched dsRNA of choice in the colonogenic assay described above are shown graphically in melanoma cells in Figure 5. The percentage of control colonies is plotted against the dose of $rI_n{}^\bullet r(C_{11-14},U)_n$ in micrograms per ml and IRU/ml for the interferons; 100% colony control is indicated as a baseline. Note both IFNs are above the baseline (no control) except at cytotoxic amounts.

Synergistic antiproliferative effect of IFN-alpha and $rI_n{}^\bullet r(C_{12},U)_n$ in human renal cell carcinoma is shown in Figure 6, again as a percentage of control colonies. Two quantities of the $rI_n{}^\bullet r(12,U)_n$ at 50 and 100 micrograms per ml in each combination were used. The concentration of IFN-alpha ranged from 0 to 3000 IRU/ml, which is well above the normal IFN-alpha therapeutic concentration.

There is a break in the line following initial readings (indicating no effect on control colonies for 0 IFN, about 62% for 50 and about 50% for 100 $rI_n{}^\bullet r(C_{12},U)_n$) then a reading for 100 IRU/ml IFN-alpha. As this data shows, IFN-alpha alone was not effective in the clinically acceptable dosage range; effectiveness was found for IFN-alpha above this, but at cycotoxic levels, levels too high for clinical use. The results of this assay indicate for effective therapy, the quantity of IFN must be reduced to a clinically acceptable level and, preferably, supplemented or replaced with another antiproliferative agent.

Specific examples of synergistic effects were seen in 63% of melanomas, 50% of breast cancers, 80% of ovarian carcinomas and 65% of renal carcinomas for combined IFN-alpha + $rI_n{}^\bullet r(C_{12},U)_n$ therapy. Thus, it can be determined from these illustrations that (a) the application of specific mismatched dsRNAs in lymphokine-resistant states will be of clinical utility; (b) in those instances of resistance to dsRNA alone, judicious combination with lymphokines should result in significant therapeutic benefit in a majority of cases.

**F. Three clinical examples confirming the efficacy of this invention are given below**

Patient 1 - malignant melanoma

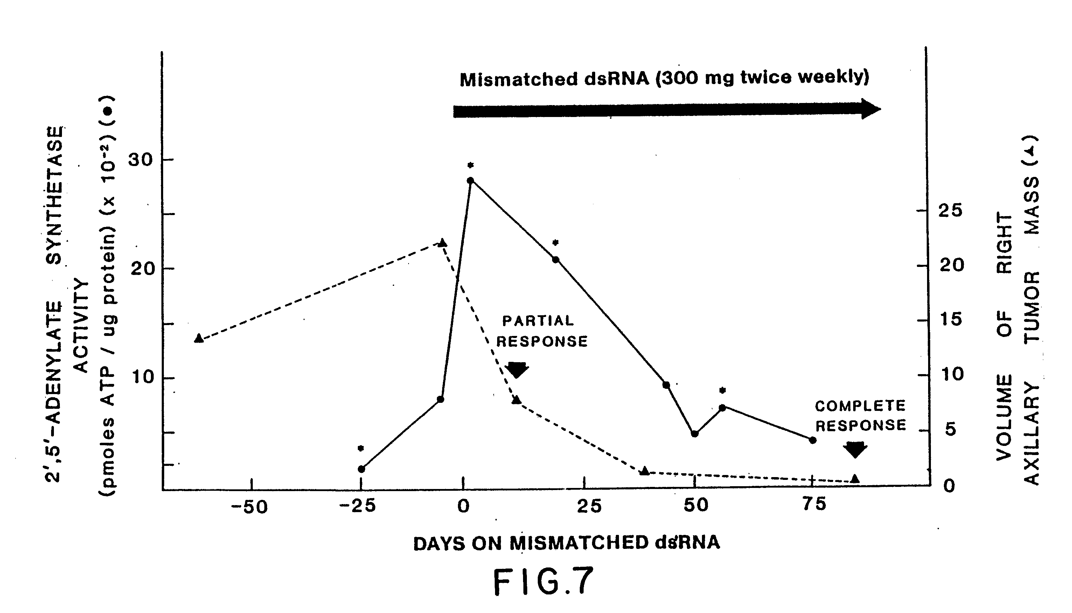

Patient 1, a middle-aged male, having malignant melanoma clinically unresponsive to IFN and IL2. Administration of dsRNA (300 mg twice weekly) resulted in complete elimination of all measurable tumor (calculated as volume of tumor mass) at the conclusion of 80 days of therapy; see Figure 7. This favorable response continues approximately 2 1/2 years from initial treatment. The present maintenance dosage is between 50 and 100 mg twice weekly. The dosage is determined by considering clinical response in light of laboratory parameters including the relative level of the $2',5'$A synthetase enzyme and the ability to detect active biochemical intermediates in the dsRNA-induced effect, such as the cyclic AMP levels described above or the presence of specific classes of $2',5'$-A molecules which underscore the tumor arrested situation as well as an enhanced immune cell profile. This is also illustrated in Figure 7, where the inventor determined nearly contemporaneous increases in $2',5'$-A synthetase, cAMP, and bioactive $2',5'$-A oligomers -- none of which were present while the patient received lymphocyte therapy or in his untreated "wash-out" period. The $2',5'$-adenylate synthetase activity (p moles ATP/$\mu$g protein) is indicated by the dark circle data points plotted against the volume of the tumor mass (indicated by the dark triangular data points) for several days prior to initiation of therapy, upon initiation of mismatched dsRNA therapy, and at the end of therapy when a complete response (virtually zero tumor volume) was observed.

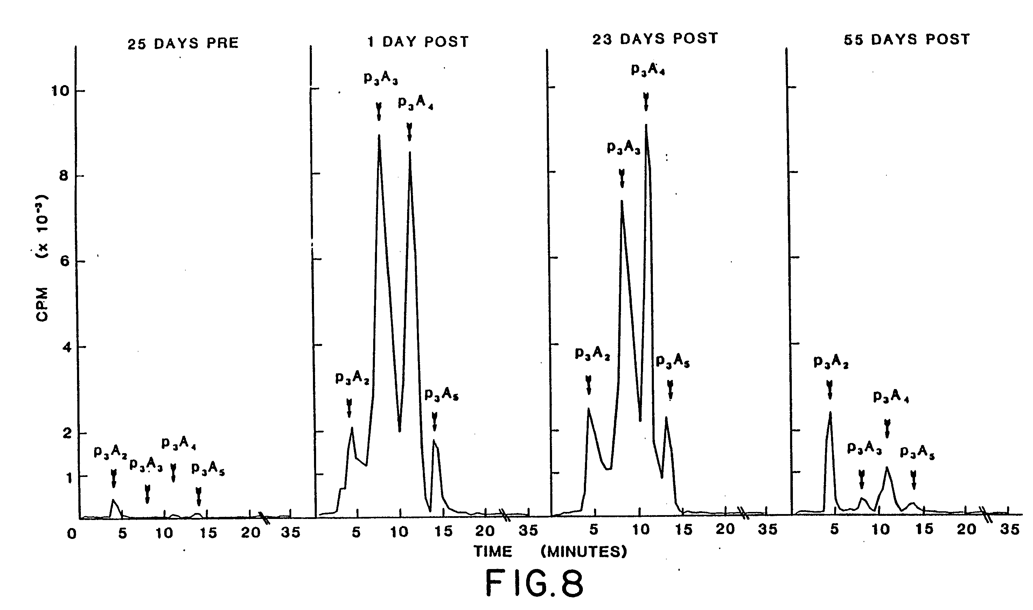

See also Figure 8 for the same patient, reporting the oligomers designated $P_3A_3$ and above (as determined by high pressure liquid chromotography) and the fact that these oligomers do not appear until after the mismatched dsRNA is administered and note the correlation of the presence of these oligomers and the clinical response. The various oligomers were measured at intervals 25 days prior to therapy with the mismatched dsRNA and 1, 23 and 55 days after initiation of therapy. Low molecular weight oligomers of $2',5'$-A (dimers) are established both in the inventor's laboratory and elsewhere to be especially bioinert with respect to antigrowth (antiproliferation), antiviral and immune enhancing properties.

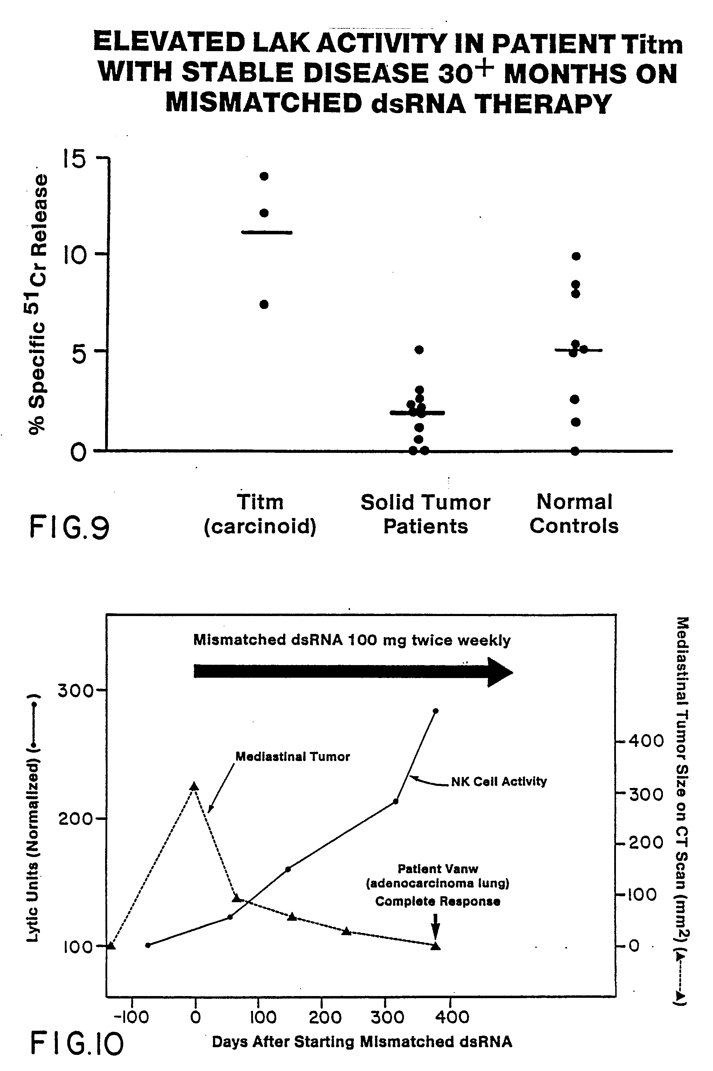

Similar biochemical phenomena underscore the prolonged elevation in lymphokine-activated killer (LAK) cell activity and the resultant stable disease and/or dramatic tumor regression as shown in Figure 9, which measures elevated LAK activity (as % specific $^{51}$Cr release) in a patient with a stabilized disease state (carcinoid cancer) following more that 30 months therapy with the dsRNA (left column) as compared with solid tumor patients not receiving dsRNA therapy (center) and normal patients or controls (right column). This data is used to monitor the relative amount of therapeutic agent required to sustain a favorable clinical effect over long periods of time, such as in maintenance therapy following initial tumor control, even in the face of lymphokine resistance.

## Patient 2

Of particular significance is the inventor's recognition that cancer spread to bone can be dramatically reduced by the combination of the dsRNA and IFN. For example, patient 2, with renal cell cancer and extensive boney metastases, obtained rapid and prolonged response after receiving a daily dose of only 1.5 m units of IFN-alpha and 300 mg of the dsRNA administered twice a week. In the instance of chronic leukemia, a similar regimen induced prolonged remissions in a high percentage of individuals whereas patients receiving IFN-alpha alone showed no significant clinical response.

## Patient 3

Patient 3, a middle-aged male with IFN-resistant kidney tumor, experienced rapid onset of tumor reduction contemporaneously with a shift in the profile of $2',5'$-A oligomers, a rapid onset of cAMP pathway derangement and a dramatic increase in immunosurveillant activity. These significant clinical and laboratory findings have persisted for about 36 continuous months and are attendant with no drug-related side effects.

Similar observations and clinical success have been obtained in a majority of lymphokine-resistant tumors, including the most lethal and indolent types of lung cancer, the data for which is summarized in Figure 10. Comparing NK cell activity (measured in lytic units and mediastinal tumor size (in mm$^2$) measured by CT scan. Results are reported before, at the initiation of, and at the completion of nearly 400 days of mismatched dsRNA therapy. The patient presented a complete clinical response to the therapy on the basis of tumor size measurements.

## Claims

1. The use of a mismatched dsRNA for the manufacture of a medicament for modulating tumour cell growth towards normalisation and loss of malignant cell phenotype.

2. The use of a mismatched dsRNA according to claim 1 wherein the dsRNA is capable of rendering dsRNA-deficient cells which are non-responsive or sub-responsive to lymphokine modulation, as evidenced by anti tumour growth activity, responsive to lymphokine modulation.

3. The use of a mismatched dsRNA according to claim 1 or 2 wherein the dsRNA has the same three-dimensional structure as dsRNA in respect of which the tumour cells are deficient.

4. The use of a $2',5'$ A synthetase-activating mismatched dsRNA for the manufacture of a medicament for improving the antigrowth activity of an interferon in non-responding or sub-responding tumour cells capable of inducing the synthesis in a tumour cell mass of a dsRNA which participates in tumour cell growth regulation in association with an interferon.

5. The use of a mismatched dsRNA according to any one of the preceding claims in which the dsRNA is constructed from oligomer complexes, polymer complexes or both, and preferably is composed of oligonucleotides hybridized with complementary single stranded polymeric RNA to form the dsRNA duplex.

6. The use of a mismatched dsRNA according to any one of the preceding claims in which the dsRNA contains regions of bond breakage and exhibits the therapeutic ratio properties of $r^I n.r(C_{11-14},^U)^n$.

7. The use of a mismatched dsRNA according to any one of claims 2 to 6 wherein the lymphokine is an interleukin or an interferon.

8. The use of a mismatched dsRNA according to any one of the prceding claims wherein the tumour cells are kidney tumour cells, malignant melanoma cells, chronic lymphocytic leukemia cells or lung tumour cells.

FIG.1A   Time (min)

FIG.1B   Time (hr)

FIG.2A

FIG.2B

FIG.3

## FIG.4

FIG.5

FIG.6

FIG.7

FIG.8

EP 0 347 501 A1

**ELEVATED LAK ACTIVITY IN PATIENT Titm
WITH STABLE DISEASE 30⁺ MONTHS ON
MISMATCHED dsRNA THERAPY**

FIG.9

FIG.10

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | EP-A-0 213 921 (HEM RESEARCH)<br>* pages 4, 5, 7 *<br>--- | 1-8 | A 61 K 31/70<br>C 07 H 21/00 |
| X | EP-A-0 013 162 (W.A. CARTER et al.)<br>* whole document *<br>--- | 1-8 | |
| Y | THE LANCET<br>6th June 1987, page 1286; W. CARTER et al.: "Clinical, immunogical and virological effects of Ampligen a mismatched double-stranded RNA, in patients with AIDS or AIDS related complex" * page 1290 *<br>--- | 1-8 | |
| Y | CHEMICAL ABSTRACTS<br>vol. 104, no. 19, 12th May 1986, abstract no. 166709, Columbus, Ohio, US; H. HUBBEL et al.: "Synergistic antiproliferative effects of human interferons in combination with mismatched double-stranded RNA on human tumor cells" & Int. J. Cancer, 1986, vol. 37, no. 3, pages 359 - 365<br>--- | 1-8 | TECHNICAL FIELDS SEARCHED (Int. Cl.4)<br><br>C 07 H<br>A 61 K |
| Y | CHEMICAL ABSTRACTS<br>vol. 104, no. 16, 21st April 1986, abstract no. 141833, Columbus, Ohio, US; SUHADOLNIK et al.: "Ampligen treatment of renal cell carcinoma: changes in 2-5A synthetase, 2-5A oligomer size and natural killer cell activity associated with antitumor response clinically." & Prog. Clin. Biol. Res., 1985, 202(2-5A syst), pages 449 - 456<br>---     -/- | 1-8 | |

The present search report has been drawn up for all claims

| Place of search | Date of completion of the search | Examiner |
|---|---|---|
| BERLIN | 19-01-1989 | AVEDIKIAN P.F. |

EPO FORM 1503 03.82 (P0401)

**European Patent Office**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | BIOLOGICAL ABSTRACTS abstract no. 33061246, May 1987, Georgia, US; H. HUBBEL et al.: "Potentiated antitumor and immunomodulatory effects of rIL-2 and mismatched double-stranded RNA Ampligen in-vivo" & Proc. Am. Assoc. Cancer Res. Annu. Meet. 1987, vol. 28, page 370 --- | 1-8 | |
| A,D | US-A-4 024 222 (P.O.P. TS'O) * whole document * ----- | 1-8 | |
| | | | TECHNICAL FIELDS SEARCHED (Int. Cl.4) |

The present search report has been drawn up for all claims

| Place of search | Date of completion of the search | Examiner |
|---|---|---|
| BERLIN | 19-01-1989 | AVEDIKIAN P.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                    

& : member of the same patent family, corresponding document

EPO FORM 1503 03.82 (P0401)